# EUROPÄISCHE PATENTSCHRIFT

(11) **EP 1 361 903 B1**
(45) Veröffentlichungstag und Bekanntmachung des Hinweises auf die Patenterteilung: **27.10.2004**
(21) Anmeldenummer: 02703599.7
(22) Anmeldetag: 18.02.2002
(51) Int. Cl.: A61L 15/00

(54) **WUNDAUFLAGE**
WOUND DRESSING
PANSEMENT

(30) Priorität: 20.02.2001 DE 10108083
(43) Veröffentlichungstag der Anmeldung: 19.11.2003
(73) Patentinhaber: Lohmann & Rauscher GmbH & Co. KG, 56567 Neuwied (DE)
(72) Erfinder: KIEL, Andrea, 3582 TM Utrecht (NL); LEUCHTEN, Elke, 47799 Krefeld (DE); WOLF, Thomas, 56203 Höhr-Grenzhausen (DE); PONTZEN, Thomas, 50737 Köln (DE)
(74) Vertreter: Patentanwälte Leinweber & Zimmermann
(86) Internationale Anmeldenummer: PCT/EP2002/001713
(87) Internationale Veröffentlichungsnummer: WO 2002/066085

(56) Entgegenhaltungen:
- EP-A- 0 053 936
- EP-A- 0 099 758
- GB-A- 2 127 389
- US-A- 4 715 857
- US-A- 4 817 594

## Beschreibung

Die Erfindung betrifft eine Wundauflage, insbesondere Wundkompresse, nach dem Oberbegriff des Patentanspruchs 1.

Derartige Wundauflagen in Form von chirurgischen Verbänden oder Tampons sind beispielsweise in der EP 0 053 936 B1 beschrieben. Bei den in dieser Schrift beschriebenen Produkten wird die aktivkohlehaltige Schicht zum Adsorbieren von Geruchsstoffen und Mikroorganismen eingesetzt und trägt daher zur Verminderung des Infektionsrisikos bei der Wundbehandlung bei. Dabei kann die Aktivkohleschicht der bekannten Wundauflage in der Form eines Aktivkohle-Stoffs vorliegen und zwischen zwei Abdeckschichten angeordnet sein. Zur weiteren Reduzierung des Infektionsrisikos ist bei dem aus der genannten Schrift bekannten Produkt ein antimikrobiell wirkendes Mittel an der aktivkohlehaltigen Schicht adsorbiert, wobei dieses antimikrobiell wirkende Mittel in einer Menge vorliegt, welche nicht mehr als 20% der adsorbierenden Stellen, die zur Adsorption des antimikrobiell wirkenden Mittels befähigt sind, absättigt. Nach der genannten Schrift kommen als antimikrobiell wirkende Mittel Jod, chlorierte Phenole, Antibiotika u. dgl. in Betracht. Der Offenbarungsgehalt der EP 0 053 936 B1 wird hinsichtlich der darin beschriebenen antimikrobiell wirkenden Mittel und aktivkohlehaltigen Schicht hiermit durch ausdrückliche Inbezugnahme in diese Beschreibung aufgenommen.

Bei dem aus der genannten Schrift bekannten Produkt hat es sich als problematisch erwiesen, daß die aktivkohlehaltige Schicht zu einem erheblichen Teil bereits mit dem antimikrobiell wirkenden Mittel belegt ist, so daß sie nicht mehr im vollen Umfang zur Adsorption von Geruchsstoffen, Bakterien o. dgl. zur Verfügung steht.

Zur Lösung dieses Problems wird in der WO 86/05970 (US 4,817,594) eine Wundauflage mit einer aktivkohlehaltigen Schicht, aber ohne antimikrobiell wirkende Mittel vorgeschlagen, bei der das Infektionsrisiko dadurch vermindert wird, daß neben der aktivkohlehaltigen Schicht auf der Außenseite der Wundauflage auch noch eine Schicht aus einem durchlässigen Material und eine Schicht aus einem halbdurchlässigen bzw. semipermeablen Material vorgesehen ist, wobei diese Schichten insgesamt antibakteriell wirkende Eigenschaften hervorbringen, indem das Wachstum der an der aktivkohlehaltigen Schicht adsorbierten Bakterien durch Begrenzung der Sauerstoffzufuhr gehemmt wird und die genannten Schichten eine Barriere für Bakterien bereitstellen, wodurch eine Kontamination von außen unterbunden wird. In der genannten Schrift wird ferner ein Verfahren zum Herstellen einer aktivkohlehaltigen Schicht beschrieben, bei dem zur Herstellung eines textilen Flächengebildes geeignete Fasern mit einer Lösung aus anorganischen Halogenen imprägniert und dann zur Aktivierung kontrolliert erwärmt werden. Der Offenbarungsgehalt wird hinsichtlich der darin beschriebenen aktivkohlehaltigen Schichten und deren Herstellung hiermit durch ausdrückliche Inbezugnahme in diese Beschreibung aufgenommen.

Weiterhin ist aus der EP 0 311 364 auch noch eine aktivkohlehaltige Schicht aufweisende Wundauflage bekannt, bei der mindestens 10 % des Porenvolumens der Aktivkohle in Form von Mesoporen vorliegt. Auch der Offenbarungsgehalt der EP 0 311 364 A2 wird hiermit durch ausdrückliche Inbezugnahme in diese Beschreibung aufgenommen.

Darüber hinaus offenbart die WO 86/05971 A1 eine Wundauflage mit einer ein antimikrobiell wirksames Mittel enthaltenden weiteren Schicht. Es handelt sich hierbei um ein elektrisch leitendes Maschengeflecht. Endlich ist aus der EP 0 691 113 A1 eine Wundauflage bekannt, bei der die Saugschicht mit einem antiseptischen Mittel imprägniert ist.

Wenngleich mit den vorstehend beschriebenen Produkten das Infektionsrisiko bei der Wundbehandlung deutlich reduziert werden kann, hat es sich gezeigt, daß auch bei Einsatz von ggf. mit einem antimikrobiell wirkenden Mittel imprägnierten aktivkohlehaltigen Schichten immer wieder Wundinfektionen auftreten.

Angesichts der vorstehend beschriebenen Probleme im Stand der Technik liegt der Erfindung die Aufgabe zugrunde, eine Wundauflage, insbesondere Wundkompresse bereitzustellen, mit der das Infektionsrisiko bei der Wundbehandlung weiter reduziert werden kann.

Erfindungsgemäß wird diese Aufgabe durch die im kennzeichnenden Teil des Anspruchs 1 angegebene Weiterbildung der beispielsweise aus der EP 0 053 936 B1 bekannten Wundauflage der eingangs beschriebenen Art gelöst.

Diese Erfindung beruht auf der überraschend einfachen Erkenntnis, daß die antimikrobiellen Eigenschaften eines antimikrobiell wirksamen Mittels auch dann noch sinnvoll eingesetzt werden, wenn dieses Mittel nicht direkt auf der die Bakterien adsorbierenden aktivkohlehaltigen Schicht aufgebracht, sondern in einer davon getrennten weiteren Schicht der Wundkompresse untergebracht ist, um so die volle Adsorptionsfähigkeit der aktivkohlehaltigen Schicht für die Adsorption von Geruchsstoffen, Bakterien u. dgl. zur Verfügung zu stetlen.

Dabei kann die das antimikrobiell wirksame Mittel enthaltende weitere Schicht in Form eines textilen Flächengebildes, insbesondere eines Faservlieses, Gewebes und/oder Gewirks vorliegen. Bei der erfindungsgemäßen Wundauflage ist das antimikrobiell wirksame Mittel in Filamenten, Fasern und/oder Gamen der Umhüllung für die aktivkohlehaltige Schicht enthalten. Wenn diese Filamente, Fasern und/oder Garne in Form von Polymeren vorliegen, kann das antimikrobiell wirksame Mittel vor und/oder während der Polymerisation in die Filamente, Fasern und/oder Garne eingebracht werden, um so Textilien mit antimikrobiellen Eigenschaften zu erhalten, die fest und nicht auswaschbar im Textil fixiert sind.

Bevorzugte Ausführungsformen der Erfindung sind in den abhängigen Ansprüchen angegeben.

Nachstehend wird die Erfindung unter Bezugnahme auf die Zeichnung, auf die hinsichtlich aller erfindungswesentlichen Arbeiten der Beschreibung nicht weiter herausgestellten Einzelheiten ausdrücklich verwiesen wird, erläutert. In der Zeichnung zeigt:

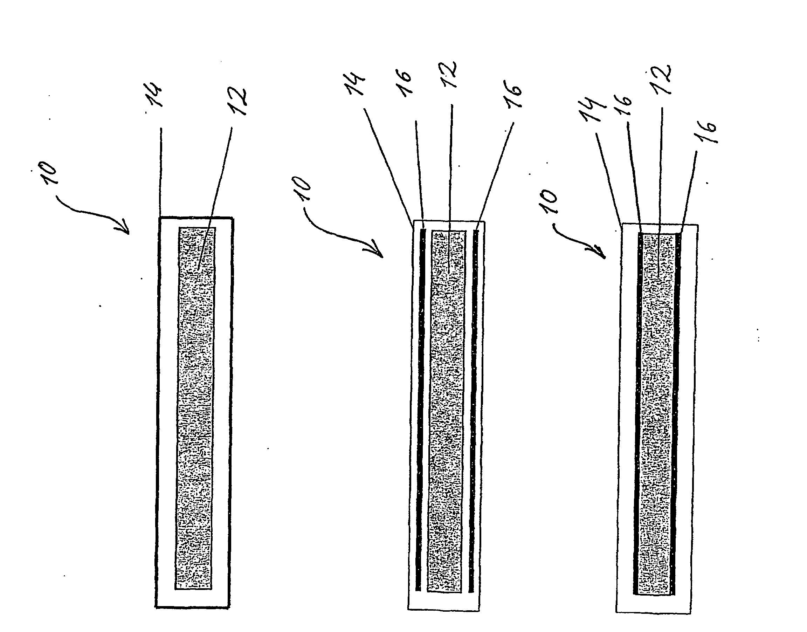

- Fig. 1: eine schematische Darstellung einer Ausführungsform der Erfindung,

Die in Fig. 1 dargestellte Wundkompresse 10 besteht im wesentlichen aus einem aktivkohlehaltigen flächigen Gebilde 12 und einer dieses Flächengebilde 12 vollständig umschließenden Umhüllung 14, die in Form eines Hüllvlieses vorliegen kann. Bei der Wundkompresse nach Fig. 1 ist das antimikrobiell wirksame Mittel in der Umhüllung 14 enthalten.

Die Erfindung ist nicht auf das anhand der Zeichnungen erläuterte Beispiel beschränkt. Vielmehr ist auch an den Einsatz von bindenförmigen Wundauflagen oder zum Tamponieren tiefer Wunden geeigneter Wundauflagen gedacht. Femer kann die erfindungsgemäße Wundauflage auch noch weitere funktionelle Schichten aufweisen.

## Patentansprüche

1. Wundauflage, insbesondere Wundkompresse (10) mit einer aktivkohlehaltigen Schicht (12) und mindestens einer weiteren, ein antimikrobiell wirksames Mittel enthaltenden Schicht, **dadurch gekennzeichnet, daß** die mindestens eine weitere Schicht eine Umhüllung (14) für die aktivkohlehaltige Schicht ist und das antimikrobiell wirksame Mittel enthaltende Filamente, Fasern und/oder Game aufweist.

2. Wundauflage nach Anspruch 1, **dadurch gekennzeichnet, daß** die Umhüllung (14) für die aktivkohlehaltige Schicht ein textiles Flächengebilde, insbesondere ein Vlies, Gewebe und/oder Gewirk, aufweist.

3. Wundauflage nach Anspruch 1 oder 2, **dadurch gekennzeichnet, daß** die Filamente, Fasern und/oder Game aus Polymeren bestehen und das antimikrobiell wirksame Mittel vor der Polymerisation in die Filamente, Fasern und/oder Game eingebracht wird.

4. Wundauflage nach einem der vorhergehenden Ansprüche, **dadurch gekennzeichnet, daß** die mindestens eine weitere Schicht eine das antimikrobiell wirksame Mittel enthaltende Folie aufweist.

5. Wundauflage nach einem der Ansprüche 3 bis 4, **dadurch gekennzeichnet, daß** die Folie und/oder das textile Flächengebilde mit der aktivkohlehaltigen Schicht und/oder einer weiteren Schicht der Wundauflage, wie etwa einer Saugschicht verbunden, insbesondere verklebt und/oder vernadelt ist.

6. Wundauflage nach einem der vorhergehenden Ansprüche, **dadurch gekennzeichnet, daß** das antimikrobiell wirksame Mittel dosiert aus der mindestens einen weiteren Schicht freisetzbar ist.

7. Wundauflage nach einem der vorhergehenden Ansprüche, **dadurch gekennzeichnet, daß** das antimikrobiell wirksame Mittel bei Oberflächenkontakt wirksam wird.

8. Wundauflage nach einem der vorhergehenden Ansprüche, **dadurch gekennzeichnet, daß** das antimikrobiell wirksame Mittel Silber und/oder Silberionen aufweist.

9. Wundauflage nach einem der vorhergehenden Ansprüche, **dadurch gekennzeichnet, daß** das antimikrobiell wirksame Mittel Silberzeolith, ein kristallines Material, wie etwa Silberzirkoniumphosphat und/oder eine glasartige Verbindung aufweist.

10. Wundauflage nach einem der vorhergehenden Ansprüche, **dadurch gekennzeichnet, dass** das antimikrobiell wirksame Mittel in einer zwischen der Umhüllungsschicht (14) und der aktivkohlehaltigen Schicht (12) angeordneten und vorzugsweise mit der aktivkohlehaltigen Schicht verbundenen Schicht (16) enthalten ist.

11. Wundauflage nach Anspruch 10 **gekennzeichnet durch** eine auf der der das antimikrobiell wirksame Mittel enthaltenden Schicht (16) abgewandten Seite der aktivkohlehaltigen Schicht (12) angeordneten Saugschicht und/oder Wäscheschutzschicht.

12. Wundauflage nach einem der vorhergehenden Ansprüche, **dadurch gekennzeichnet, dass** das antimikrobiell wirksame Mittel Chitin, Chitosan, Trichlosan und/oder Chlorhexidin aufweist.

## Claims

1. Wound dressing, in particular wound compress (10) with an activated charcoal-containing layer (12) and at least one further layer containing an antimicrobially active agent, **characterised in that** the at least one further layer is a covering (14) for the activated charcoal-containing layer and has filaments, fibres and/or yarns containing the antimicrobially active agent.

2. Wound dressing according to claim 1, **characterised in that** the covering (14) for the activated charcoal-containing layer has a textile surface structure, in particular a non-woven fleece, woven fabric and/or knitted fabric.

3. Wound dressing according to claim 1 or 2, **characterised in that** the filaments, fibres and/or yarns comprise polymers and the antimicrobially active agent is introduced into the filaments, fibres and/or yarns before the polymerisation.

4. Wound dressing according to one of the previous claims, **characterised in that** the at least one further layer has a film containing the antimicrobially active agent.

5. Wound dressing according to one of the claims 3 or 4, **characterised in that** the film and/or the textile surface structure is connected to the activated charcoal-containing layer and/or a further layer of the wound dressing, such as an absorptive layer, in particular glued and/or needle bonded.

6. Wound dressing according to one of the previous claims, **characterised in that** the antimicrobially active agent is releasable in dosed manner from the at least one further layer.

7. Wound dressing according to one of the previous claims, **characterised in that** the antimicrobially active agent becomes effective on surface contact.

8. Wound dressing according to one of the previous claims, **characterised in that** the antimicrobially active agent has silver and/or silver ions.

9. Wound dressing according to one of the previous claims, **characterised in that** the antimicrobially active agent has silver zeolite, a crystalline material, such as silver zirconium phosphate and/or a glass-like compound.

10. Wound dressing according to one of the previous claims, **characterised in that** the antimicrobially active agent is contained in a layer (16) arranged between the covering layer (14) and the activated charcoal-containing layer (12) and preferably connected to the activated charcoal-containing layer.

11. Wound dressing according to claim 10, **characterised in that** it has an absorptive layer and/or a washproof layer on the side of the activated charcoal-containing layer (12) facing away from the layer (16) containing the antimicrobially active agent.

12. Wound dressing according to one of the previous claims, **characterised in that** the antimicrobially active agent has chitin, chitosan, trichlosan and/or chlorhexidine.

## Revendications

1. Pansement, notamment compresse (10), avec une couche (12) contenant du charbon actif et avec au moins une autre couche contenant un agent antimicrobien, **caractérisé en ce que** la, au moins une, autre couche, est une enveloppe (14) pour la couche contenant du charbon actif et présente des filaments, fibres et/ou fils contenant l'agent antimicrobien.

2. Pansement selon la revendication 1, **caractérisé en ce que** l'enveloppe (14) pour la couche contenant du charbon actif présente un produit textile formant surface, notamment une étoffe non-tissée, un tissu et/ou un tissu à mailles.

3. Pansement selon la revendication 1 ou 2,
**caractérisé en ce que** les filaments, fibres et/ou fils sont en polymères et l'agent antimicrobien est introduit avant la polymérisation dans les filaments, fibres et/ou fils.

4. Pansement selon l'une des revendications précédentes, **caractérisé en ce que** la, au moins une, autre couche, présente une feuille contenant l'agent antimicrobien.

5. Pansement selon l'une des revendications 3 à 4, **caractérisé en ce que** la feuille et/ou le produit textile formant surface sont assemblés, notamment collés et/ou aiguilletés, à la couche contenant du charbon actif et/ou à une autre couche du pansement, par exemple une couche absorbante.

6. Pansement selon l'une des revendications précédentes, **caractérisé en ce que** l'agent antimicrobien peut se dégager, dosé, de l'autre ou des autres couches.

7. Pansement selon l'une des revendications précédentes, **caractérisé en ce que** l'agent antimicrobien est efficace lors d'un contact de surfaces.

8. Pansement selon l'une des revendications précédentes, **caractérisé en ce que** l'agent antimicrobien présente de l'argent et/ou des ions argent.

9. Pansement selon l'une des revendications précédentes, **caractérisé en ce que** l'agent antimicrobien présente de la zéolite d'argent, un matériau cristallin, par exemple du phosphate de zirconium-argent et/ou une liaison du type verre.

10. Pansement selon l'une des revendications précédentes, **caractérisé en ce que** l'agent antimicrobien est contenu dans une couche (16) qui est placée entre la couche (14) formant enveloppe et la couche (12) contenant du charbon actif et qui est assemblée de préférence à la couche contenant du charbon actif.

11. Pansement selon la revendication 10, **caractérisé par** une couche absorbante et/ou une couche protégeant le linge qui est placée sur celui des côtés de la couche (12) contenant du charbon actif qui est à l'opposé de la couche (16) contenant l'agent antimicrobien.

12. Pansement selon l'une des revendications précédentes, **caractérisé en ce que** l'agent antimicrobien présente de la chitine, du chitosane, du trichlosan et/ou de la chlorhexidine.
